# EUROPÄISCHE PATENTSCHRIFT

(11) **EP 2 241 262 B1**
(45) Veröffentlichungstag und Bekanntmachung des Hinweises auf die Patenterteilung: **19.09.2018**
(21) Anmeldenummer: 10158282.3
(22) Anmeldetag: 30.03.2010
(51) Int. Cl.: A61B 17/02

(54) **Chirurgische Retraktionsvorrichtung**
Surgical retraction device
Dispositif de rétraction chirurgical

(30) Priorität: 16.04.2009 DE 102009018139
(43) Veröffentlichungstag der Anmeldung: 20.10.2010
(73) Patentinhaber: Aesculap AG, 78532 Tuttlingen (DE)
(72) Erfinder: Rothweiler, Christoph, 78166, Donaueschingen (DE); Morales, Pedro, 78532, Tuttlingen (DE); Nesper, Markus, 78532, Tuttlingen (DE); Weißhaupt, Dieter, 78194, Immendingen (DE)
(74) Vertreter: Hoeger, Stellrecht & Partner Patentanwälte mbB

(56) Entgegenhaltungen:
- US-A1- 2004 225 196
- US-A1- 2005 277 812
- US-A1- 2006 271 096

## Beschreibung

Die vorliegende Erfindung betrifft eine chirurgische Retraktionsvorrichtung mit mindestens zwei relativ zueinander bewegbaren und direkt oder indirekt aneinander gehaltenen Haltearmen, an denen jeweils mindestens ein chirurgisches Rückhalteelement zum Rückhalten von Knochen oder Knochengewebe angeordnet oder anordenbar ist, und mit einer Haltearmpositioniereinrichtung zum Bewegen und Ändern einer Stellung der mindestens zwei Haltearme relativ zueinander, wobei die Haltearmpositioniereinrichtung eine Grobjustageeinrichtung und mindestens eine Feinjustageeinrichtung umfasst, mit denen eine Stellung der mindestens zwei Haltearme relativ zueinander unabhängig voneinander änderbar ist, wobei die Retraktionsvorrichtung einen Grundkörper umfasst, an welchem mindestens einer der mindestens zwei Haltearme bewegbar gehalten ist, und wobei die mindestens eine Feinjustageeinrichtung in Form einer ersten Feinantriebseinrichtung ausgebildet ist zum Bewegen eines der mindestens zwei Haltearme und des Grundkörpers relativ zueinander.

Chirurgische Retraktionsvorrichtungen mit mindestens zwei relativ zueinander bewegbaren und direkt oder indirekt aneinander gehaltenen Haltearmen, an denen jeweils mindestens ein chirurgisches Rückhalteelement zum Rückhalten von Knochen oder Knochengewebe angeordnet oder anordenbar ist, und mit einer Haltearmpositioniereinrichtung zum Bewegen und Ändern einer Stellung der mindestens zwei Haltearme relativ zueinander kommen in der Chirurgie, beispielsweise in der Herz-Thorax-Chirurgie, zum Einsatz. Sie werden in Form sogenannter selbsthaltender Sperrer verwendet, um einen übersichtlichen Zugang zu den zu behandelnden Organen zu bekommen. Ein Zugang zum Brustkorb eines Patienten erfolgt dabei entweder zwischen zwei Rippen hindurch oder durch das zuvor geteilte Sternum. Die Sperrer müssen dabei großen Kräften standhalten, um den Zugang zum Operationssitus offen zu halten. Damit die zum Freihalten des Operationssitus erforderlichen Kräfte vom Bedienpersonal, beispielsweise einem Operateur, problemlos aufgebracht werden können und die Retraktionsvorrichtungen auch in der Lage sind, diese Kräfte aufzunehmen, sind die Retraktionsvorrichtungen mit einer Haltearmpositioniereinrichtung ausgestattet, um eine Stellung der mindestens zwei Haltearme relativ zueinander zu ändern, indem mindestens einer derselben bewegt wird. Bei bekannten Haltearmpositioniereinrichtungen ist eine Taktilität derselben jedoch üblicherweise beschränkt aufgrund der Konstruktion derselben.
Chirurgische Retraktionsvorrichtungen sind insbesondere aus der US 2007/ 0161865 A1 und der US 2007/0073111 A1 bekannt. Ferner sind aus der US 2005/0277812 A1, der US 2006/0271096 A1 sowie der US 2004/0225196 A1 weitere chirurgische Retraktionsvorrichtungen bekannt.
Es ist daher eine Aufgabe der vorliegenden Erfindung, eine chirurgische Retraktionsvorrichtung der eingangs beschriebenen Art so zu verbessern, dass deren Aufbau besonders einfach wird.
Diese Aufgabe wird bei einer chirurgischen Retraktionsvorrichtung der eingangs beschriebenen Art erfindungsgemäß dadurch gelöst, dass die erste Feinantriebseinrichtung in Form eines Feinzahnstangenantriebs ausgebildet ist.
Eine derart ausgebildete Haltearmpositioniereinrichtung ermöglicht ein besonders feinfühliges Einstellen eines Abstands oder einer bestimmten Stellung zwischen den mindestens zwei Haltearmen. Durch die unabhängige oder getrennte Bedienbarkeit der Grobjustageeinrichtung und der mindestens einen Feinjustageeinrichtung können Stellungen der mindestens zwei Haltearme, häufig sind es bei derartigen chirurgischen Retraktionsvorrichtungen genau zwei Haltearme, mit der Grobjustageeinrichtung vorpositioniert und mit der Feinjustageeinrichtung dosiert und gefühlvoll in die gewünschte Endstellung gebracht werden. Durch die herkömmlichen relativ groben Haltearmpositioniereinrichtungen geht eine Rückkopplung beziehungsweise das Gefühl zwischen den zu spreizenden Geweben beziehungsweise Knochen und dem Anwender nahezu verloren, so dass es teilweise zu Sternum- und/oder Rippenbrüchen kommt, wenn eine Bedienperson mit der nicht sehr feinfühlig betätigbaren Haltearmpositioniereinrichtung einen Abstand der mindestens zwei Haltearme relativ zueinander etwas zu groß wählt. Diese Probleme können bei der erfindungsgemäß weitergebildeten chirurgischen Retraktionsvorrichtung nicht mehr auftreten, da mit der mindestens einen Feinjustageeinrichtung, nach einer Grobpositionierung der mindestens zwei Haltearme relativ zueinander mittels der Grobjustageeinrichtung, eine endgültige Positionierung der Haltearme in feinfühliger Weise möglich ist. Eine Konstruktion der chirurgischen Retraktionsvorrichtung wird insbesondere dadurch vereinfacht, dass sie einen Grundkörper umfasst, an welchem mindestens einer der mindestens zwei Haltearme bewegbar gehalten ist. Gemäß der Erfindung ist vorteilhafterweise vorgesehen, dass die mindestens eine Feinjustageeinrichtung in Form einer ersten Feinantriebseinrichtung ausgebildet ist zum Bewegen eines der mindestens zwei Haltearme und des Grundkörpers relativ zueinander. Die erste Feinantriebseinrichtung ermöglicht ein gefühlvolles und dosiertes Positionieren der mindestens zwei Haltearme relativ zueinander. Besonders einfach wird der Aufbau der chirurgischen Retraktionsvorrichtung dadurch, dass die erste Feinantriebseinrichtung in Form eines Feinzahnstangenantriebs ausgebildet ist.

Besonders einfach wird der Aufbau der chirurgischen Retraktionsvorrichtung, wenn die Grobjustageeinrichtung und/oder die mindestens eine Feinjustageeinrichtung ausgebildet sind zum Bewegen der mindestens zwei Haltearme parallel zueinander aufeinander zu und/oder voneinander weg. Beispielsweise können die mindestens zwei Haltearme bei derartigen Justageeinrichtungen relativ zueinander verschoben werden.

Des Weiteren kann es alternativ oder ergänzend vorteilhaft sein, wenn die Grobjustageeinrichtung und/oder die mindestens eine Feinjustageeinrichtung ausgebildet sind zum Verschwenken der mindestens zwei Haltearme um mindestens eine Schwenkachse aufeinander zu und/oder voneinander weg. Beispielsweise kann die Grobjustageeinrichtung ausgebildet sein in Form einer Verschiebeeinrichtung für die mindestens zwei Haltearme, die mindestens eine Feinjustageeinrichtung in Form einer Verschiebe- oder Verschwenkeinrichtung.

Insbesondere dann, wenn die Retraktionsvorrichtung zwei Haltearme umfasst, ist es günstig, wenn sie auch zwei Feinjustageeinrichtungen umfasst. Dies gestattet es, die Position jedes Haltearms gefühlvoll und dosiert durch eine Bedienperson zu ändern.

Vorzugsweise sind zwei Haltearme am Grundkörper bewegbar gehalten. Es können jedoch auch mehr Haltearme am Grundkörper bewegbar gehalten sein. Die bewegbar am Grundkörper gehaltenen Haltearme können dann individuell mit der Haltearmpositioniereinrichtung relativ zueinander und auch zum Grundkörper bewegt und positioniert werden.

Besonders einfach handhaben lässt sich die chirurgische Retraktionsvorrichtung, wenn ein Haltearm am Grundkörper feststehend angeordnet ist. Eine Abstands- oder Positionsänderung der mindestens zwei Haltearme relativ zueinander erfolgt in diesem Fall durch die Änderung einer Position eines weiteren Haltearms relativ zum Grundkörper.

Gemäß einer bevorzugten Ausführungsform der Erfindung kann vorgesehen sein, dass die Grobjustageeinrichtung eine Grobantriebseinrichtung umfasst zum Bewegen eines der mindestens zwei Haltearme und des Grundkörpers relativ zueinander. Mit der Grobjustageeinrichtung kann einer der Haltearme relativ zum Grundkörper beziehungsweise zu einem weiteren Haltearm grob positioniert werden.

Besonders einfach wird der Aufbau der chirurgischen Retraktionsvorrichtung, wenn die Antriebseinrichtung in Form eines Grobzahnstangenantriebs ausgebildet ist.

Günstig ist es, wenn der Grobzahnstangenantrieb eine Grobzahnreihe am Grundkörper und ein am Grundkörper verschiebbar gelagertes Groblagerelement umfasst, wenn am Groblagerelement ein verzahntes Grobantriebsglied bewegbar gelagert ist, welches mit der Grobzahnreihe in Eingriff steht, und wenn infolge einer Bewegung des Grobantriebsglieds relativ zum Groblagerelement das Groblagerelement relativ zum Grundkörper bewegbar ist. Mit einem derart aufgebauten Grobzahnstangenantrieb kann auf einfache Weise ein am Groblagerelement feststehend oder bewegbar gehaltener Haltearme relativ zum Grundkörper bewegt werden.

Der Aufbau der chirurgischen Retraktionsvorrichtung kann weiter vereinfacht werden, wenn das Grobantriebsglied in Form eines Grobzahnrads ausgebildet ist. Es ist insbesondere auf eine Verzahnung der Grobzahnreihe abgestimmt.

Vorteilhafterweise ist das Grobantriebsglied um eine Grobantriebsgliedrotationsachse am Groblagerelement rotierbar gelagert, welche quer zu einer Längsachse des Grundkörpers verläuft. Eine Längsachse des Grundköpers kann insbesondere auch eine Längsachse eines Grundkörperabschnitts sein, wenn der Grundkörper nicht geradlinig ausgebildet ist. In jedem Fall kann durch die beschriebene Anordnung durch einfache Rotation des Grobantriebsglieds das Groblagerelement parallel zur Längsachse des Grundkörpers bewegt werden.

Um die Grobjustageeinrichtung auf einfache Weise betätigen zu können, ist es vorteilhaft, wenn sie ein Grobbetätigungselement zum Bewegen des Grobantriebsglieds relativ zum Groblagerelement umfasst.

Günstig ist es, wenn der Feinzahnstangenantrieb eine Feinzahnreihe am Grundkörper und ein am Grundkörper verschiebbar gelagertes Feinlagerelement umfasst, wenn am Feinlagerelement ein verzahntes Feinantriebsglied bewegbar gelagert ist, welches mit der Feinzahnreihe in Eingriff steht, und wenn infolge einer Bewegung des Feinantriebsglieds relativ zum Feinlagerelement das Feinlagerelement relativ zum Grundkörper bewegbar ist. Die Feinzahnreihe weist günstigerweise eine Zahnteilung auf, die einem vielfachen einer Zahnteilung der Grobzahnreihe entspricht, beispielsweise dem zwei- bis fünffachen. Dies ermöglicht es umgekehrt, um das zwei- bis fünffache feiner einen Abstand beziehungsweise eine Relativposition zwischen den mindestens zwei Haltearmen einzustellen.

Vorteilhafterweise ist das Feinantriebsglied in Form eines Feinzahnrads ausgebildet.

Vorzugsweise ist das Feinantriebsglied um eine Feinantriebsgliedrotationsachse am Feinlagerelement rotierbar gelagert, welche quer zu einer Längsachse des Grundkörpers verläuft. Diese Anordnung ermöglicht es, durch einfache Rotation des Feinantriebsglieds um die Feinantriebsgliedrotationsachse das Feinlagerelement, an dem ein Haltearm feststehend oder bewegbar angeordnet sein kann, parallel zur Längsachse des Grundkörpers zu bewegen.

Um die erste Feinantriebseinrichtung einfach und sicher betätigen zu können, ist es vorteilhaft, wenn die chirurgische Retraktionsvorrichtung ein Feinbetätigungselement zum Bewegen des Feinantriebsglieds relativ zum Feinlagerelement umfasst.

Vorteilhafterweise erstrecken sich die Grobzahnreihe und/oder die Feinzahnreihe jeweils bis zu einem freien Ende des Grundkörpers. Dies gestattet es auf einfache Weise, das Feinlagerelement und das Groblagerelement vom Grundkörper zu trennen und wieder mit diesem in Eingriff zu bringen. Die Grobzahnreihe und die Feinzahnreihe können in derselben Seitenfläche des Grundkörpers ausgebildet sein, jedoch auch in unterschiedlichen Seitenflächen. Insbesondere ist es denkbar, dass die Grobzahnreihe und die Feinzahnreihe derart am Grundkörper ausgebildet sind, dass sie in unterschiedliche Richtungen voneinander weg weisen. Sie können ferner auch am gleichen Abschnitt des Grundkörpers angeordnet oder ausgebildet sein.

Gemäß einer weiteren bevorzugten Ausführungsform der Erfindung kann vorgesehen sein, dass die mindestens eine Feinjustageeinrichtung in Form einer zweiten Feinantriebseinrichtung ausgebildet ist zum Verschwenken eines Haltearms relativ zum Grundkörper. Insbesondere kann der Haltearm an einem Feinlagerelement angeordnet sein, welches relativ zum Grundkörper feststehend oder beweglich ausgebildet sein kann. Die zweite Feinantriebseinrichtung ermöglicht es auf einfache Weise, den mindestens einen Haltearm relativ zum Grundkörper in definierter Weise und feinfühlig zu verschwenken.

Besonders einfach wird der Aufbau der chirurgischen Retraktionsvorrichtung, wenn die zweite Feinantriebseinrichtung am Groblagerelement oder am Grundkörper angeordnet oder ausgebildet ist. Selbstverständlich ist es auch denkbar, die zweite Feinantriebseinrichtung an einem separaten Lagerelement anzuordnen, welches am Grundkörper bewegbar gehalten ist. Wie bereits dargelegt, könnte ein Lagerelement der zweiten Feinantriebseinrichtung auch feststehend am Grundkörper angeordnet oder ausgebildet sein.

Auf einfache Weise verschwenken lässt sich der mindestens eine Haltearm wenn die zweite Feinantriebseinrichtung einen Feinspindeltrieb umfasst. Eine Rotationsbewegung des Feinspindeltriebs oder eines Teils derselben kann so auf einfache Weise in eine Verschwenkbewegung des mindestens einen Haltearms relativ zum Grundkörper umgesetzt werden.

Der Aufbau des Feinspindeltriebs wird besonders einfach, wenn er eine Antriebsspindel umfasst, welche mit einem Haltearm oder dem Groblagerelement in Kontakt steht, und wenn infolge einer Bewegung der Antriebspindel der Haltearm relativ zum Grundkörper um eine Schwenkachse verschwenkbar ist. Beispielsweise kann ein distales Ende der Antriebsspindel am Haltearm oder an einem Vorsprung desselben anliegen oder angreifen, mit diesem in Eingriff stehen oder gekoppelt sein, und zwar derart, dass infolge einer Vorschubbewegung der Antriebsspindel aufgrund einer Rotation derselben eine definierte Verschwenkbewegung des Haltearms relativ zum Grundkörper erfolgt.

Günstigerweise ist die Antriebsspindel in einer Antriebsspindelführung verschraubbar geführt. So kann die Antriebsspindel um ihre Längsachse rotiert und dadurch relativ zur Antriebsspindelführung eine Schraubenbewegung ausführen.

Besonders einfach wird der Aufbau der chirurgischen Retraktionsvorrichtung, wenn die Antriebsspindelführung am Grundkörper angeordnet oder ausgebildet ist. Es sind dann keine weiteren Lagerelemente erforderlich, um den Feinspindelantrieb auszubilden.

Vorzugsweise ist die Antriebsspindelführung am Groblagerelement oder am Grundkörper angeordnet oder ausgebildet. Diese Konstruktion ermöglicht es, einen am Groblagerelement gelagerten Haltearm grob zu verstellen mittels der Grobjustageeinrichtung sowie fein einzustellen mittels der am Groblagerelement oder am Grundkörper angeordneten zweiten Feinantriebseinrichtung.

Um den mindestens einen Haltearm in kleinen Schritten und feinfühlig in seiner Stellung relativ zum Grundkörper zu ändern, kann es günstig sein, wenn die Antriebsspindel ein Feingewinde umfasst.

Vorteilhaft ist es, wenn die chirurgische Retraktionsvorrichtung ferner eine Anschlageinrichtung zum Begrenzen einer Bewegung der mindestens zwei Haltearme relativ zueinander aufweist. So kann sichergestellt werden, dass die Haltearme nicht völlig unkontrolliert bewegt werden. Insbesondere bei einem herz- oder thoraxchirurgischen Eingriff könnte dies für einen Patienten negative Folgen haben.

Um sicherzustellen, dass die mindestens zwei Haltearme in einer gewünschten Position verbleiben können, ist es vorteilhaft, wenn die chirurgische Retraktionsvorrichtung eine Feststelleinrichtung zum Festlegen der mindestens zwei in einer Justagestellung relativ zueinander bewegbaren Haltearme in einer Rückhaltestellung relativ zueinander umfasst. Die in der Justagestellung in ihrer Position veränderbaren mindestens zwei Haltearme können nach Erreichen der von der Bedienperson gewünschten Relativstellung relativ zueinander festgelegt werden, so dass die Retraktionsvorrichtung dann die Rückhaltestellung einnimmt.

Je nach Art des durchzuführenden chirurgischen Eingriffs kann es günstig sein, wenn die Haltearme geradlinig oder gekrümmt ausgebildet sind.

Die nachfolgende Beschreibung bevorzugter Ausführungsformen der Erfindung dient im Zusammenhang mit der Zeichnung der näheren Erläuterung. Es zeigen:
- Figur 1:: eine schematische Darstellung einer chirurgischen Retraktionsvorrichtung beim Spreizen eines durchtrennten Sternums eines Patienten;
- Figur 2:: eine perspektivische Ansicht der in Figur 1 dargestellten Retraktionsvorrichtung;
- Figur 3:: eine Explosionsdarstellung der Retraktionsvorrichtung aus Figur 2;
- Figur 4A:: eine Schnittansicht längs Linie 4-4 in Figur 3;
- Figur 4B:: eine Schnittansicht längs Linie 4-4 in Figur 3;
- Figur 4C:: eine vergrößerte Darstellung des Endbereichs der Retraktionsvorrichtung aus Figur 2;
- Figur 5:: eine perspektivische Ansicht eines weiteren, nicht erfindungsgemäßen Ausführungsbeispiels einer Retraktionsvorrichtung mit einem feststehenden Haltearm;
- Figur 6:: eine weitere perspektivische Ansicht der in Figur 5 dargestellten Retraktionsvorrichtung mit einem verschwenkten Haltearm; und
- Figur 7A:: eine Seitenansicht eines weiteren, nicht erfindungsgemäßen Ausführungsbeispiels einer Retraktionsvorrichtung;
- Figur 7B:: eine teilweise durchbrochene Seitenansicht des vorderen Endbereichs der Retraktionsvorrichtung.

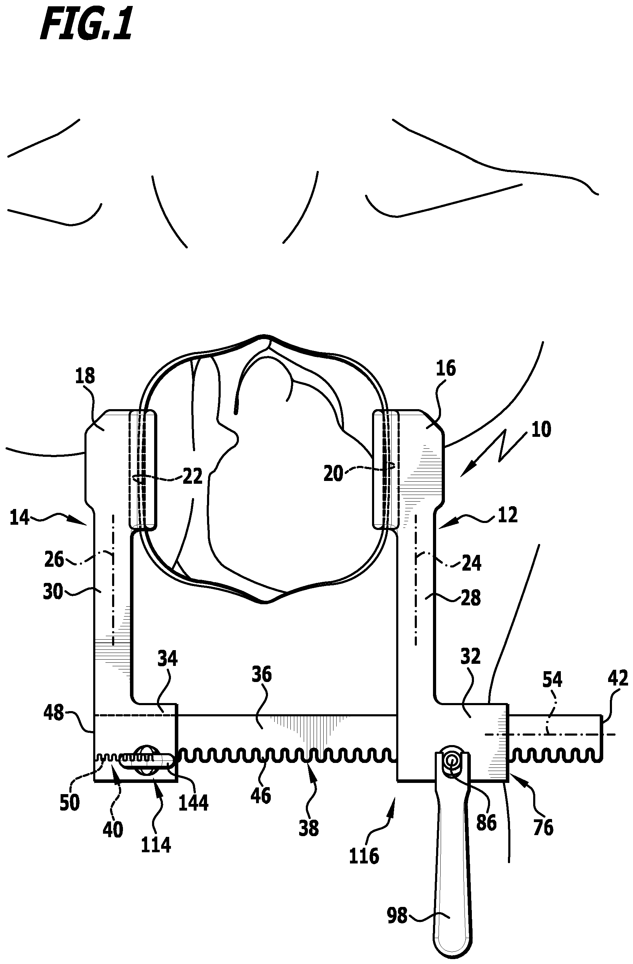

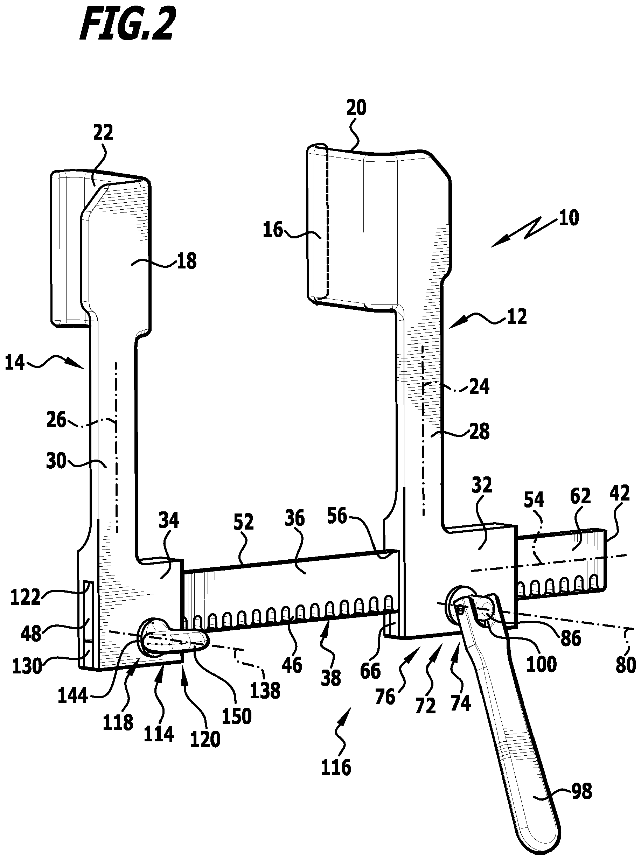

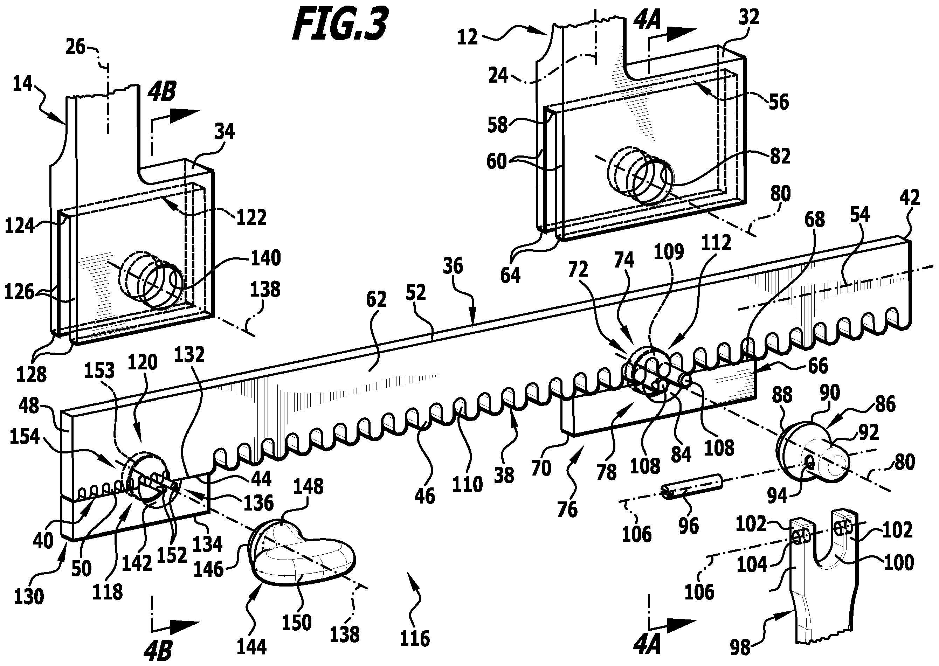

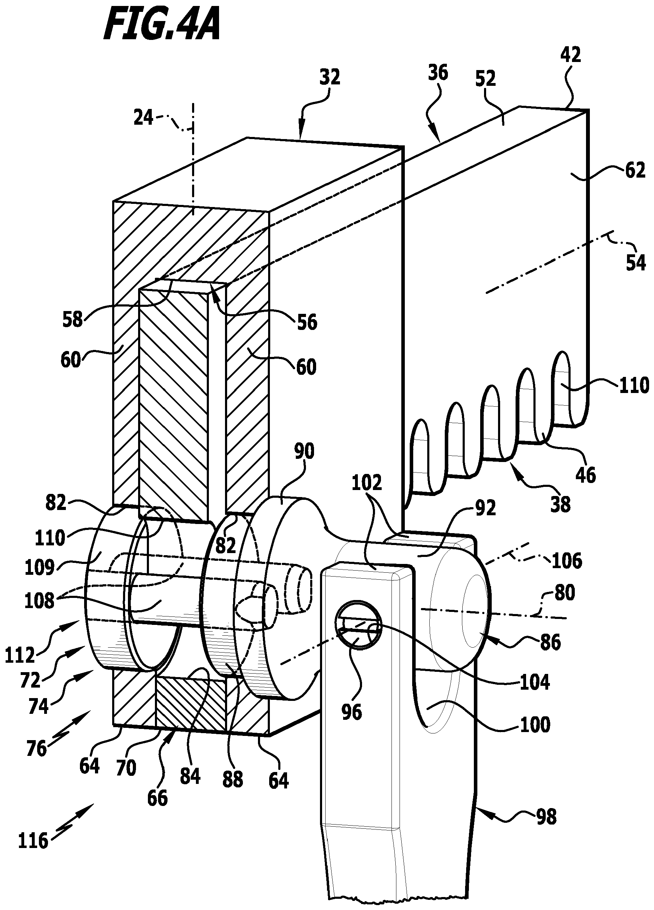

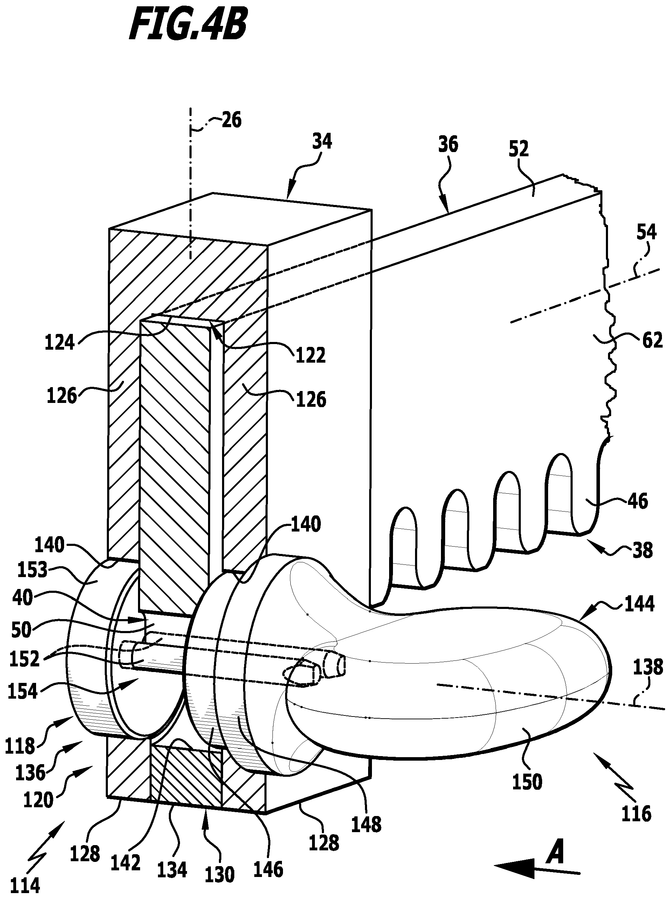

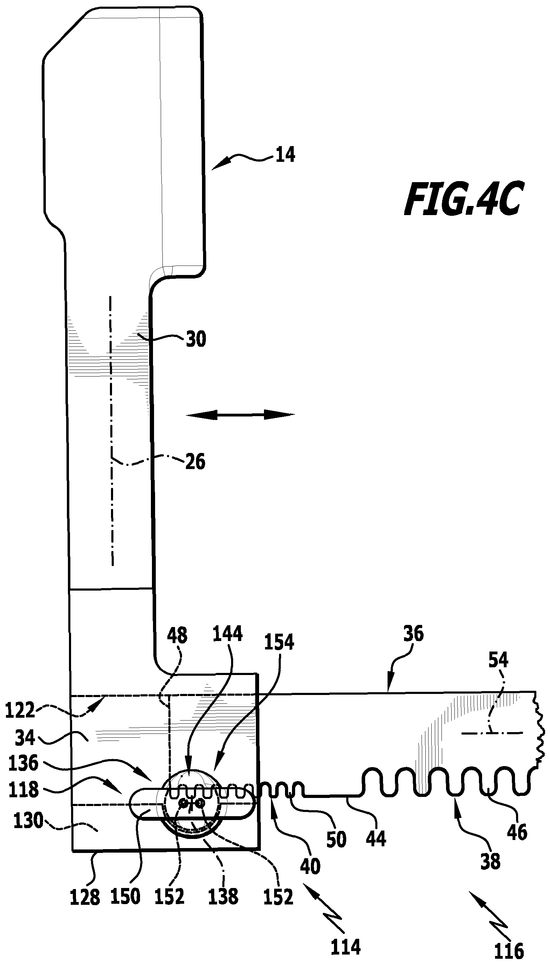

Ein erstes Ausführungsbeispiel einer chirurgischen Retraktionsvorrichtung, die nachfolgend auch als Sperrer bezeichnet wird, ist in den Figuren 1 bis 4 insgesamt mit dem Bezugszeichen 10 bezeichnet. Sie umfasst zwei relativ zueinander bewegbare und indirekt aneinander gehaltene Haltearme 12 und 14, an denen jeweils ein chirurgisches Rückhalteelement 16 beziehungsweise 18 einstückig angeformt ist. Optional wäre es denkbar, Rückhalteelemente auch derart auszubilden, dass sie mit den Haltearmen 12 und 14 lösbar verbindbar sind. Die Rückhalteelemente 16 und 18 weisen jeweils eine im Wesentlichen nutförmige Aufnahme 20 beziehungsweise 22 auf, in die eine Rippe oder eine Hälfte eines durchtrennten Sternums, wie in Figur 1 dargestellt, eingreifen und so durch die Rückhalteelemente 16 und 18 zurückgehalten werden können.

Die Haltearme 12 und 14 definieren jeweils Längsachsen 24 beziehungsweise 26 die parallel zueinander verlaufen. Sie werden im Wesentlichen definiert durch einen flachen quaderförmigen Halteabschnitt 28 beziehungsweise 30, die an einem Ende das Rückhalteelement 16 beziehungsweise 18 tragen und an ihrem anderen Ende jeweils ein Lagerelement, und zwar der Lagerarm 12 ein Groblagerelement 32 und der Halteabschnitt 30 eine Feinlagerelement 34. Das Groblagerelement 32 und das Feinlagerelement 34 sind beide beweglich an einem Grundkörper 36 der chirurgischen Retraktionsvorrichtung 10 bewegbar gehalten, wobei der Grundkörper 36 in Form einer quaderförmigen Stange oder Schiene ausgebildet ist.

Der Grundkörper 36 ist mit zwei Zahnungen versehen, die eine Grobzahnreihe 38 und eine Feinzahnreihe 40 definieren. Die Grobzahnreihe 38 ist ausgehend von einem ersten Ende 42 über etwa dreiviertel einer Gesamtlänge des Grundkörpers 36 auf einer schmalen Seitenkante 44 ausgebildet und umfasst senkrecht abstehende, ausgerundete Zähne 46. Ausgehend vom anderen Ende 48 des Grundkörpers erstreckt sich die Feinzahnreihe 40 mit einer Mehrzahl von Zähnen 50, die in derselben Seitenkante 44 ausgebildet sind. Eine sich parallel zur Seitenkante 44 erstreckende schmale Seitenkante 52 des Grundkörpers 36 erstreckt sich auch parallel zu einer vom Grundkörper 36 definierten Längsachse 54.

Das Groblagerelement 32 ist in Form eines Quaders ausgebildet und mit einer nutförmigen Aufnahme 76 versehen, die eine Breite aufweist, welche einer Breite des Grundkörpers 36 entspricht. Ein Boden 58 der Aufnahme 56 liegt an der Seitenkante 52 an. Die Aufnahme 56 seitlich begrenzende Seitenwände 60 und 62 liegen an die Seitenkanten 44 und 52 miteinander verbindenden Seitenflächen 62 an und ragen mit ihren freien Stirnflächen 64 über die Seitenkante 44 vor.

Die Aufnahme 56 ist mit einem quaderförmigen Verschlusskörper 66 verschlossen, der eine dem Groblagerelement 32 parallel zur Längsachse 54 entsprechende Länge aufweist. Eine Oberseite 68 des Verschlusskörpers 66 liegt an den Zähnen 46 an, eine Unterseite 70 des Verschlusskörpers 66 schließt bündig mit den Stirnflächen 64 ab. Das Groblagerelement 32 ist so definiert geführt parallel zur Längsachse 54 auf dem Grundkörper 36 verschiebbar.

Zum definierten Bewegen des Groblagerelements 32 auf dem Grundkörper 36 dient eine Grobantriebseinrichtung 72, die zum Bewegen des Haltearms 12 und des Grundkörpers 36 relativ zueinander und in Form eines Grobzahnstangenantriebs 74 ausgebildet ist. Die Grobantriebseinrichtung 72 bildet einen Teil einer Grobjustageeinrichtung 76, mit der eine Stellung der Haltearme 12 und 14 relativ zueinander änderbar ist. Der Grobzahnstangenantrieb 74 umfasst die Grobzahnreihe 38, das Groblagerelement 32 und ein verzahntes Grobantriebsglied 78, welches mit der Grobzahnreihe 38 in Eingriff steht. Es ist um eine Grobantriebsgliedrotationsachse 80 am Groblagerelement 32 rotierbar gelagert, welche quer zur Längsachse 54 des Grundkörpers 36 verläuft. Das Groblagerelement 32 ist mit einer Querbohrung 82 versehen, die die beiden Seitenwände 60 durchsetzt und derart angeordnet ist, dass im Verschlusskörper 66 eine in Richtung auf die Grobzahnreihe 38 weisende Vertiefung 84 ausgebildet wird, die die Form eines halben Hohlzylinders aufweist.

Ein Drehkörper 86 weist ein erstes Ende auf mit einem flachen zylindrischen Abschnitt 88, welcher formschlüssig in die Querbohrung 82 in einer Seitenwand 60 einsetzbar ist und nicht in die Aufnahme 56 hineinragt. An den Abschnitt 88 schließt sich ein zylindrischer Anschlagkörper 90 an, welcher einen Außendurchmesser aufweist, der etwas größer ist als die Querbohrung 82. Ein Endabschnitt 92, der sich an den Anschlagkörper 90 anschließt, weist einen Durchmesser auf, der noch etwas kleiner ist als der Durchmesser des Abschnitts 88. Er ist zudem quer zur Grobantriebsgliedrotationsachse 80 mit einer Bohrung 94 versehen, die zur Aufnahme eines Lagerstifts 96 dient, welcher beidseitig über den Endabschnitt 92 vorsteht. Er dient zum beweglichen Kuppeln mit einem in Form eines Betätigungshebels ausgebildeten Grobbetätigungselement 98, welches ein U-förmig ausgebildetes Ende 100 mit zwei parallelen Lagerschenkeln 102, zwischen die der Endabschnitt 92 eingreift, aufweist, die beide jeweils mit einer Bohrung 104 versehen sind, in die der Lagerstift 96 drehfest eingesetzt ist. Ein Außendurchmesser des Lagerstifts 96 ist etwas kleiner als ein Innendurchmesser der Bohrung 94, so dass der Lagerstift 96 und das an diesem festgelegte Grobbetätigungselement 98 um eine von der Bohrung 94 definierte Längsachse 106 verschwenkbar sind.

Vom Abschnitt 88 stehen vom Anschlagkörper 90 weg weisend und parallel zur Grobantriebsgliedrotationsachse 80 diametral einander gegenüberliegend zwei Zahnstifte 108 ab in einem Abstand, der einem Abstand der Zähne 46 entspricht, so dass die Zahnstifte 108 in zwei benachbarte Zahnzwischenräume 110 eingreifen können. In die Querbohrung 82 der anderen Seitenwand 60 ist eine Lagerscheibe 109 formschlüssig eingesetzt, in der die Zahnstifte 108 gesichert sind. Durch Rotation des Drehkörpers 86 kämmt das in Form eines Grobzahnrads 112 ausgebildete Grobantriebselement 78, welches den Abschnitt 88 und die Zahnstifte 108 umfasst, mit der Grobzahnreihe 38, so dass das Groblagerelement 32 und damit der Haltearm 12 entweder in Richtung auf das Ende 42 hin bewegt wird bei einer Rotation des Grobbetätigungselements 98 um die Grobantriebsgliedrotationsachse 80 im Gegenuhrzeigersinn beziehungsweise bei einer Rotation im Uhrzeigersinn in Richtung auf das Ende 48 hin.

Die Retraktionsvorrichtung 10 umfasst ferner eine Feinjustageeinrichtung 114, die zusammen mit der Grobjustageeinrichtung 76 eine Haltearmpositioniereinrichtung 116 bildet. Die Feinjustageeinrichtung 114 ist ausgebildet zum Ändern einer Stellung des Haltearms 14 relativ zum Grundkörper 36. Mit der Grobjustageeinrichtung 76 sowie mit der Feinjustageeinrichtung 114 können die zwei Haltearme 12 und 14 relativ zueinander und unabhängig voneinander bewegt und somit eine relative Stellung derselben verändert werden.

Die Feinjustageeinrichtung 114 ist in Form einer ersten Feinantriebseinrichtung 118 ausgebildet zum Bewegen des Haltearms 14 und des Grundkörpers 16 relativ zueinander. Sie ist in Form eines Feinzahnstangenantriebs 120 ausgebildet, der die Feinzahnreihe 40 und das Feinlagerelement 34 umfasst.

Das Feinlagerelement 34 ist in Form eines Quaders ausgebildet mit einer nutförmigen Aufnahme 122, welche einen Boden 124 aufweist, der an der Seitenkante 52 anliegt. Die Aufnahme 122 wird seitlich begrenzt durch zwei parallele Seitenwände 126, die an den Seitenflächen 62 des Grundkörpers 36 anliegen.

Die Seitenwände 126 an freien Enden begrenzende Stirnflächen 128 ragen über die Seitenkante 44 vor. Ein quaderförmiger Verschlusskörper 130 liegt mit seiner Oberseite 132 an der Seitenkante 44 an und schließt mit seiner Unterseite 134 bündig mit den Stirnflächen 128 ab. Er ist in die Aufnahme 122 eingesetzt, so dass das Feinlagerelement 34 parallel zur Längsachse 54 auf dem Grundkörper 36 geführt verschoben werden kann.

Der Feinzahnstangenantrieb 120 umfasst ferner ein Feinantriebsglied 136, welches um eine Feinantriebsgliedrotationsachse 138 am Feinlagerelement 34 rotierbar gelagert ist. Sie wird definiert durch eine Querbohrung 140, die die beiden Seitenwände 126 durchsetzt. Die Querbohrung 140 bildet ferner eine Vertiefung 142 im Verschlusskörper 130 aus, die in Richtung auf die Seitenkante 44 hin weisend geöffnet ist und die Form eines halben Hohlzylinders aufweist. Das Feinantriebsglied 136 umfasst einen Drehkörper 144, mit einem flachen zylindrischen Abschnitt 146, der formschlüssig in die Querbohrung 140 in einer Seitenwand 126 eingreift. Der Abschnitt 146 ragt nicht in die Aufnahme 122 hinein. An ihn schließt sich ein zylindrischer Anschlagkörper 148 an, dessen Außendurchmesser etwas größer ist als ein Durchmesser der Querbohrung 140. Ein im Wesentlichen quaderförmiges Feinbetätigungselement 150 ist an den Anschlagkörper 148 angeformt und bildet eine Art Betätigungsknopf für das Feinantriebsglied 136. Dieses umfasst ferner zwei Zahnstifte 152, die vom Abschnitt 146 parallel zueinander und zur Feinantriebsgliedrotationsachse 138 senkrecht vom Abschnitt 146 abstehend angeordnet sind. Die Zahnstifte 152 sind in einem Abstand voneinander angeordnet, der einem Abstand der Zähne 50 entspricht. Sie sind bezogen auf die Feinantriebsgliedrotationsachse 138 einander diametral gegenüberliegend angeordnet. Damit ist es möglich, dass die Zahnstifte 152, die zusammen mit dem Abschnitt 156 ein Feinzahnrad 154 ausbilden, mit den Zähnen 50 in Eingriff bringbar sind und mit diesen kämmen können. In der die andere Seitenwand 126 durchsetzenden Querbohrung 140 ist eine weitere Lagerscheibe 153 formschlüssig eingesetzt, an der die Zahnstifte 152 zusätzlich gesichert sind. Eine Rotation des Feinbetätigungselements 150 im Uhrzeigersinn bewegt das Feinlagerelement 34 in Richtung auf das Ende 48 hin, eine Rotation des Feinbetätigungselements 150 im Gegenuhrzeigersinn führt zu einer Verschiebebewegung des Feinlagerelements 34 in Richtung auf das Ende 42 hin.

Ein Operateur kann, wie beispielsweise in Figur 1 dargestellt, nach Durchtrennen des Sternums die Retraktionsvorrichtung 10 derart in den Schnitt einsetzen, dass die Aufnahmen 20 und 22 der Rückhalteelemente 16 und 18 jeweils einen Teil des durchtrennten Sternums aufnehmen. Durch Rotation des Betätigungselements 98 im Gegenuhrzeigersinn können die Rückhalteelemente 16 und 18 voneinander weg bewegt werden. Eine grobe Einstellung ist somit mit der Grobjustageeinrichtung 76 möglich.

Der Abstand der Zähne 50 ist etwa halb so groß wie ein Abstand der Zähne 46. Damit führt volle Umdrehung des Feinbetätigungselements 150 um die Feinantriebsgliedrotationsachse 138 nur zum halben Vorschub des Feinlagerelements 34 verglichen mit einem Vorschub des Groblagerelements 32 bei einer vollen Umdrehung des Grobbetätigungselements 98 um die Grobantriebsgliedrotationsachse 80. Ein Operateur kann damit mittels der Feinjustageeinrichtung 114 einen Abstand zwischen den Haltearmen 12 und 14 und damit zwischen den Rückhalteelementen 16 und 18 deutlich feinfühliger Dosieren als nur mit der Grobjustageeinrichtung 76 allein.

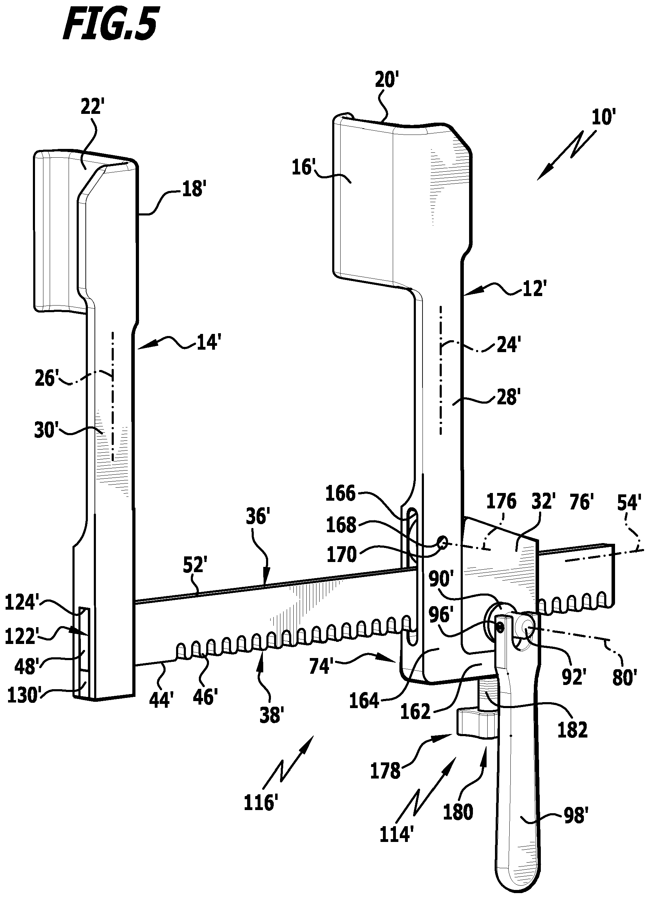

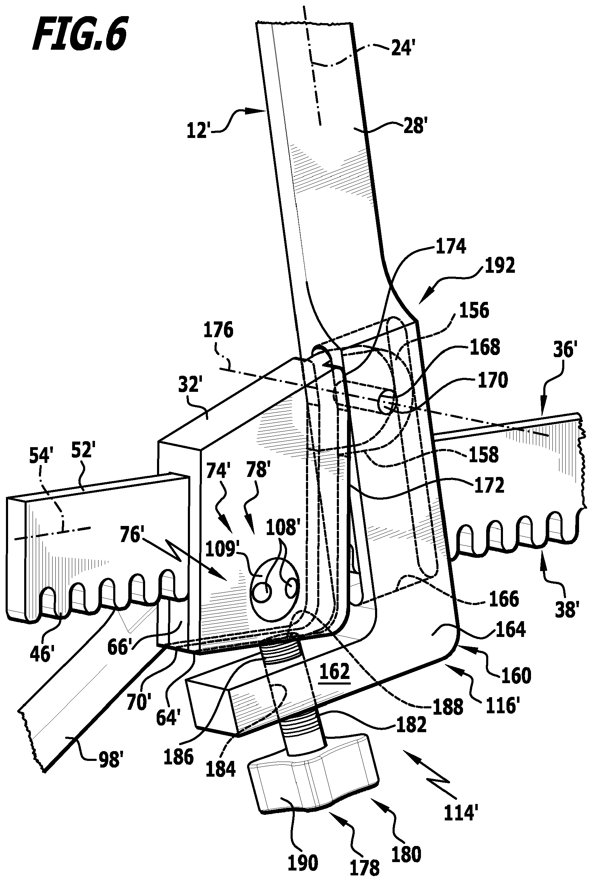

Ein weiteres Ausführungsbeispiel einer insgesamt mit dem Bezugszeichen 10' bezeichneten chirurgischen Retraktionsvorrichtung ist in den Figuren 5 und 6 schematisch dargestellt. Teile der Retraktionsvorrichtung 10', die identisch oder in ihrer Funktion mit Teilen der Retraktionsvorrichtung 10 entsprechend ausgebildet sind, werden nachfolgend und in den Figuren mit identischen Bezugszeichen mit einem zusätzlichen Anstrich bezeichnet.

Der Grundkörper 36' ist analog dem Grundkörper 36 ausgebildet, allerdings weist er lediglich eine Grobzahnreihe 38' mit Zähnen 46' auf. Am Ende 48' des Grundkörpers 36' ist der Haltearm 14' mit seiner Längsachse 26' senkrecht zur Längsachse 54' feststehend angeordnet. Hierzu ist an einem Ende des Haltearms 14' eine Aufnahme 122' ausgebildet analog der Aufnahme 122. Ein Verschlusskörper 130' verschließt die Aufnahme 122' und liegt an der Seitenkante 44' des Grundkörpers 36' an, ein Boden 124' der Aufnahme 122' an der Seitenkante 52' des Grundkörpers 36'.

Auf dem Grundkörper 36' verschiebbar parallel zur Längsachse 54' ist das quaderförmige Groblagerelement 32' gehalten. Es kann mittels der Grobjustageeinrichtung 76' analog der Grobjustageeinrichtung 76 bewegt werden.

Eine Feinjustageeinrichtung 114' ist, anders als bei der Retraktionsvorrichtung 10, nicht direkt am Grundkörper 36' gelagert, sondern am Groblagerelement 32'. Dieses weist zu diesem Zweck einen parallel zur Längsachse 54' vom Groblagerelement 32' abstehenden Vorsprung auf, welcher mit seiner Unterseite 158 an der Seitenkante 52' anliegt. Ein im Wesentlichen L-förmiges Ende 160 des Haltearms 12' umfasst einen um etwa 90° bezogen zur Längsachse 24' abstehenden Schenkel 162. Ein sich parallel zur Längsachse 24' erstreckender Abschnitt des L-förmigen Ende 160 ist mit einem Führungsschlitz 166 versehen, welcher eine Breite aufweist, die einer Breite des Grundkörpers 36' entspricht, so dass der Grundkörper 36' den Führungsschlitz 166 durchsetzen kann. Der Abschnitt 164 ist im Bereich des Führungsschlitzes 166 mit einer Bohrung 168 durchsetzt, die sich auch durch den Lagervorsprung 156 erstreckt. Der Abschnitt 164 ist mit dem Lagervorsprung 156 gekoppelt mittels eines Lagerstifts 170, der drehfest mit dem Abschnitt 164 verbunden ist und einen Außendurchmesser aufweist, welcher etwas kleiner als die den Lagervorsprung 156 durchsetzende Bohrung 168 ist.

In einer Grundstellung liegt der Schenkel 162 an den Stirnflächen 64' des Groblagerelements 32' an, der Abschnitt 164 an einer in Richtung auf das Ende 48' hin weisenden Seitenfläche 172 des Groblagerelements 32'. Im Bereich des Lagervorsprungs 156 ist eine Anschlagfläche 174 ausgebildet, die relativ zu einer von der Seitenfläche 172 definierten Ebene, die senkrecht zur Längsachse 54' orientiert ist, um circa 5° bis 15° geneigt ist. Wird der Haltearm 12' um eine vom Lagerstift 170 definierte Schwenkachse 176 verschwenkt, so ist dies nur so lange möglich, bis der Abschnitt 164 an der Anschlagfläche 174 anschlägt.

Zum definierten Verschwenken des Haltearms 12' relativ zum Grundkörper 36' ist die Feinjustageeinrichtung 114' in Form einer zweiten Feinantriebseinrichtung 178 ausgebildet. Sie ist am Groblagerelement 32' angeordnet und umfasst einen Feinspindeltrieb 180. Der Feinspindeltrieb 180 umfasst eine Antriebsspindel 182, die in einer Antriebsspindelführung 184 in Form einer mit einem Innengewinde versehenen Bohrung am Schenkel 162 ausgebildet ist. Die Antriebsspindel 182 ist mit einem Außengewinde in Form eines Feingewindes 186 versehen, welches korrespondierend zum Innengewinde der am Schenkel 162 ausgebildeten Bohrung ausgebildet ist. Ein freies Ende 188 der Antriebsspindel 182 liegt an der Unterseite 70' des Verschlusskörpers 66' an. Am anderen Ende ist an der Antriebsspindel 182 ein Drehknopf 190 angeformt, welcher ein Feinbetätigungselement bildet. Je weiter die Antriebsspindel 182 im Uhrzeigersinn in die Antriebsspindelführung 184 hineingeschraubt wird, umso weiter steht das Ende 188 von dieser vor, wodurch der Haltearm 12' um die Schwenkachse 176 verschwenkt wird, bis der Abschnitt 164 an der Anschlagfläche 174 anschlägt. Die Retraktionsvorrichtung 10' umfasst somit auch eine Anschlageinrichtung 192' zum Begrenzen einer Bewegung des Haltearms 12' relativ zum Haltearm 14' beziehungsweise zum Grundkörper 36'. Die Antriebsspindelführung 184 ist folglich am Haltearm 12' ausgebildet. Alternativ kann sie auch am Groblagerelement 32' angeordnet oder ausgebildet sein. Mit der Feinjustageeinrichtung 114' können die Haltearme 12' und 14' relativ zueinander verschwenkt werden zum kontinuierlichen Vergrößern eines Abstands zwischen den Rückhalteelementen 16' und 18'.

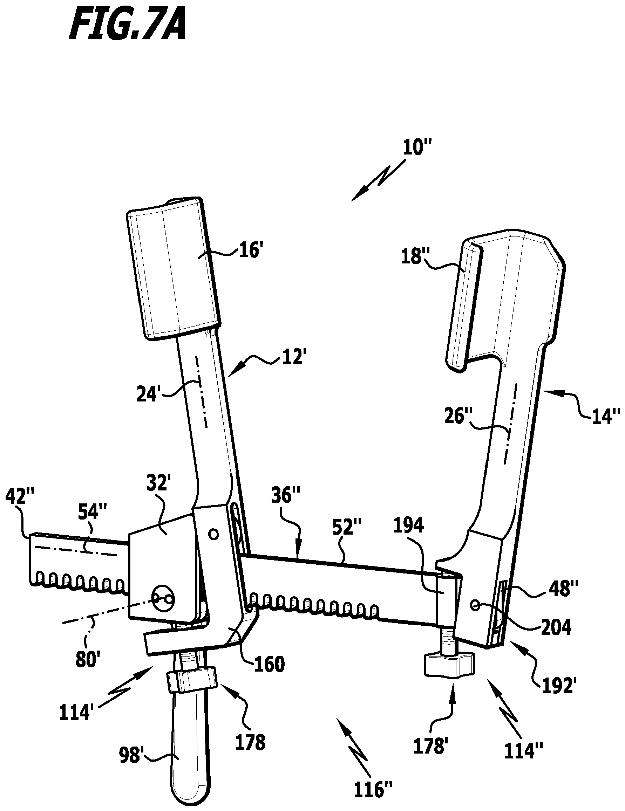

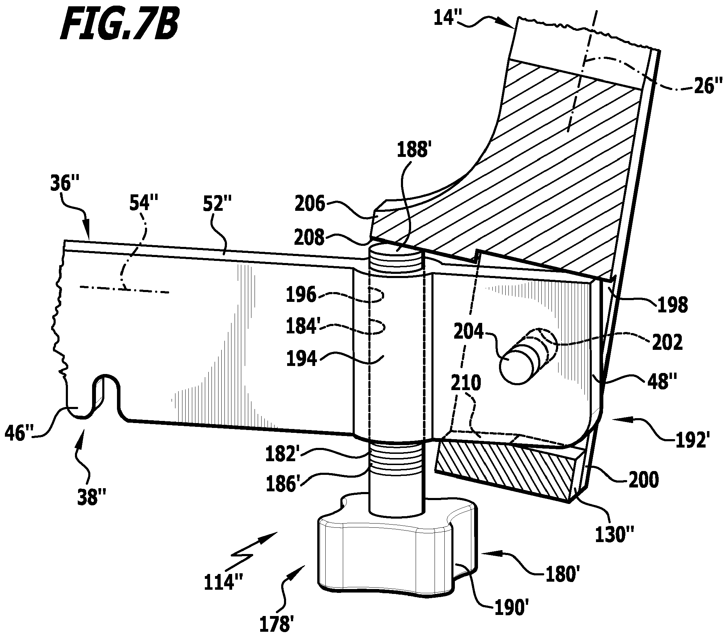

Ein weiteres Ausführungsbeispiel einer insgesamt mit dem Bezugszeichen 10" bezeichneten Retraktionsvorrichtung ist in Figur 7 schematisch dargestellt. Sie unterscheidet sich von der Retraktionsvorrichtung 10' im Wesentlichen nur dadurch, dass der Haltearm 14" relativ zum Grundkörper 36" verschwenkt werden kann mittels einer weiteren Feinjustageeinrichtung 114". Diese umfasst eine zweite Feinantriebseinrichtung 178" mit einem Feinspindeltrieb 180'. Der Feinspindeltrieb 180' umfasst eine Antriebsspindelführung 184', die in Form einer am Grundkörper 36" ausgebildeten Hülse 194 geformt ist, die ein Innengewinde 196 aufweist. Sie dient zur Führung einer Antriebsspindel 182' mit einem zum Innengewinde 196 korrespondierenden Feingewinde 186'. An einem Ende der Antriebsspindel 182' ist ein Drehknopf 190' ausgebildet, mit dem die Antriebsspindel 182' in die Antriebsspindelführung 184' hinein- oder herausgeschraubt werden kann.

Das Ende 48" greift in eine Nut 198 des freien Endes 200 des Haltearms 14" ein. Der Haltearm 14" ist im Bereich der Nut 198 ebenso wie diese mit einer Bohrung 202 versehen, in die ein Lagerstift 204 eingesetzt ist, dessen Längsachse senkrecht zur Längsachse 54" des Grundkörpers 36" orientiert ist. Oberhalb der Seitenkante 52" steht vom Haltearm 14" ein Vorsprung 206 ab, der in Richtung auf das Ende 42" hin weist. Das Ende 188' der Antriebsspindel 182' steht im Kontakt mit dem Vorsprung 206. Eine Anschlageinrichtung 192' wird gebildet durch einen Boden 208 der Nut 198 in Verbindung mit einer oberen Anschlagfläche 210 des Verschlusskörpers 130".

Wird die Antriebsspindel 182 im Uhrzeigersinn in die Antriebsspindelführung 184 hineingeschraubt, drückt das Ende 188' gegen den Vorsprung 206, wodurch der Haltearm 14" um eine Schwenkachse 212, die von der Längsachse des Lagerstifts 204 definiert wird, verschwenkt wird, bis die Seitenfläche 210 am Boden 202 anschlägt.

Bei der Retraktionsvorrichtung 10" sind somit zwei Feinjustageeinrichtungen 114' und 114" vorgesehen, die ein Verschwenken der Haltearme 14" und 12' unabhängig voneinander relativ zum Grundkörper 36" ermöglichen.

## Patentansprüche

1. Chirurgische Retraktionsvorrichtung (10) mit mindestens zwei relativ zueinander bewegbaren und direkt oder indirekt aneinander gehaltenen Haltearmen (12, 14), an denen jeweils mindestens ein chirurgisches Rückhalteelement (16, 18) zum Rückhalten von Knochen oder Körpergewebe angeordnet oder anordenbar ist, und mit einer Haltearmpositioniereinrichtung (116) zum Bewegen und Ändern einer Stellung der mindestens zwei Haltearme (12, 14) relativ zueinander, wobei die Haltearmpositioniereinrichtung (116) eine Grobjustageeinrichtung (76) und mindestens eine Feinjustageeinrichtung (114) umfasst, mit denen eine Stellung der mindestens zwei Haltearme (12, 14) relativ zueinander unabhängig voneinander änderbar ist, wobei die Retraktionsvorrichtung einen Grundkörper (36) umfasst, an welchem mindestens einer der mindestens zwei Haltearme (12, 14) bewegbar gehalten ist, und wobei die mindestens eine Feinjustageeinrichtung (114) in Form einer ersten Feinantriebseinrichtung (118) ausgebildet ist zum Bewegen eines der mindestens zwei Haltearme (12, 14) und des Grundkörpers (36) relativ zueinander, **dadurch gekennzeichnet, dass** die erste Feinantriebseinrichtung (118) in Form eines Feinzahnstangenantriebs (120) ausgebildet ist.

2. Chirurgische Retraktionsvorrichtung nach Anspruch 1, **dadurch gekennzeichnet, dass** die Grobjustageinrichtung (76) und/oder die mindestens eine Feinjustageeinrichtung (114) ausgebildet sind zum Bewegen der mindestens zwei Haltearme (12, 14) parallel zueinander aufeinander zu oder voneinander weg.

3. Chirurgische Retraktionsvorrichtung nach Anspruch 1 oder 2, **dadurch gekennzeichnet, dass** die Grobjustageinrichtung und/oder die mindestens eine Feinjustageeinrichtung ausgebildet sind zum Verschwenken der mindestens zwei Haltearme um mindestens eine Schwenkachse aufeinander zu und/oder voneinander weg.

4. Chirurgische Retraktionsvorrichtung nach einem der voranstehenden Ansprüche, **dadurch gekennzeichnet, dass** zwei oder mehr Haltearme am Grundkörper bewegbar gehalten sind.

5. Chirurgische Retraktionsvorrichtung nach einem der voranstehenden Ansprüche, **dadurch gekennzeichnet, dass** die Grobjustageeinrichtung eine Grobantriebseinrichtung (72) umfasst zum Bewegen eines der mindestens zwei Haltearme (12, 14) und des Grundkörpers (36) relativ zueinander.

6. Chirurgische Retraktionsvorrichtung nach einem der voranstehenden Ansprüche, **dadurch gekennzeichnet, dass** der Feinzahnstangenantrieb (120) eine Feinzahnreihe (40) am Grundkörper (36) und ein am Grundkörper (36) verschiebbar gelagertes Feinlagerelement (34) umfasst, dass am Feinlagerelement (34) ein verzahntes Feinantriebsglied (136) bewegbar gelagert ist, welches mit der Feinzahnreihe (40) in Eingriff steht, und dass infolge einer Bewegung des Feinantriebsglieds (136) relativ zum Feinlagerelement (34) das Feinlagerelement (34) relativ zum Grundkörper (36) bewegbar ist.

7. Chirurgische Retraktionsvorrichtung nach Anspruch 6, **dadurch gekennzeichnet, dass** das Feinantriebsglied (136) in Form eines Feinzahnrads (154) ausgebildet ist.

8. Chirurgische Retraktionsvorrichtung nach Anspruch 6 oder 7, **dadurch gekennzeichnet, dass** das Feinantriebsglied (136) um eine Feinantriebsgliedrotationsachse (138) am Feinlagerelement (34) rotierbar gelagert ist, welche quer zu einer Längsachse (54) des Grundkörpers (36) verläuft
und/oder
dass die Retraktionsvorrichtung ein Feinbetätigungselement (150) zum Bewegen des Feinantriebsglieds (136) relativ zum Feinlagerelement (34) umfasst
und/oder
dass die Grobzahnreihe (38) und/oder die Feinzahnreihe (40) sich jeweils bis zu einem freien Ende (42, 48) des Grundkörpers (36) erstrecken.

9. Chirurgische Retraktionsvorrichtung nach einem der voranstehenden Ansprüche, **dadurch gekennzeichnet, dass** die mindestens eine Feinjustageeinrichtung in Form einer zweiten Feinantriebseinrichtung ausgebildet ist zum Verschwenken eines Haltearms relativ zum Grundkörper.

10. Chirurgische Retraktionsvorrichtung nach Anspruch 9, **dadurch gekennzeichnet, dass** die zweite Feinantriebseinrichtung (178) am Groblagerelement oder am Grundkörper angeordnet oder ausgebildet ist.

11. Chirurgische Retraktionsvorrichtung nach Anspruch 9 oder 10, **dadurch gekennzeichnet, dass** die zweite Feinantriebseinrichtung (178) einen Feinspindeltrieb (180) umfasst.

12. Chirurgische Retraktionsvorrichtung nach Anspruch 11, **dadurch gekennzeichnet, dass** der Feinspindeltrieb (180; 180') eine Antriebsspindel (182) umfasst, welche mit einem Haltearm oder dem Groblagerelement in Kontakt steht, und dass infolge einer Bewegung der Antriebsspindel (182) der Haltearm relativ zum Grundkörper um eine Schwenkachse verschwenkbar ist.

13. Chirurgische Retraktionsvorrichtung nach einem der voranstehenden Ansprüche, **gekennzeichnet durch** eine Anschlageinrichtung (192) zum Begrenzen einer Bewegung der mindestens zwei Haltearme relativ zueinander.

14. Chirurgische Retraktionsvorrichtung nach einem der voranstehenden Ansprüche, **gekennzeichnet durch** eine Feststelleinrichtung zum Festlegen der mindestens zwei in einer Justagestellung relativ zueinander bewegbaren Haltearme (12, 14) in einer Rückhaltestellung relativ zueinander.

15. Chirurgische Retraktionsvorrichtung nach einem der voranstehenden Ansprüche, **dadurch gekennzeichnet, dass** die Haltearme (12, 14) geradlinig oder gekrümmt ausgebildet sind.

## Claims

1. Surgical retraction device (10) comprising at least two support arms (12, 14) which are moveable relative to each other and are held directly or indirectly upon one another, at least one surgical restraining element (16, 18) for holding back bones or body tissue which is or can be arranged on said arms, and comprising a support arm positioning mechanism (116) for moving and changing a position of the at least two support arms (12, 14) relative to each other, wherein the support arm positioning mechanism (116) comprises a coarse adjustment mechanism (76) and at least one fine adjustment mechanism (114) with which the position of the at least two support arms (12, 14) relative to each other can be changed independently of one another, wherein the retraction device comprises a base body (36) on which at least one of the at least two support arms (12, 14) is held in a moveable manner, and wherein the at least one fine adjustment mechanism (114) is constructed in the form of a first fine drive mechanism (118) for moving one of the at least two support arms (12, 14) and the base body (36) relative to each other, **characterized in that** the first fine drive mechanism (118) is constructed in the form of a fine rack gear drive (120).

2. Surgical retraction device in accordance with claim 1, **characterized in that** the coarse adjustment mechanism (76) and/or the at least one fine adjustment mechanism (114) are configured to move the at least two support arms (12, 14) in parallel with each other towards one another or away from one another.

3. Surgical retraction device in accordance with claim 1 or 2, **characterized in that** the coarse adjustment mechanism and/or the at least one fine adjustment mechanism are configured to pivot the at least two support arms about at least one pivotal axis towards one another and/or away from one another.

4. Surgical retraction device in accordance with any one of the preceding claims, **characterized in that** two or more support arms are held on the base body in moveable manner.

5. Surgical retraction device in accordance with any one of the preceding claims, **characterized in that** the coarse adjustment mechanism comprises a coarse drive mechanism (72) for moving one of the at least two support arms (12, 14) and the base body (36) relative to each other.

6. Surgical retraction device in accordance with any one of the preceding claims, **characterized in that** the fine rack gear drive (120) comprises a row of fine teeth (40) on the base body (36) and a fine bearing element (34) which is mounted on the base body (36) in displaceable manner, **in that** a toothed fine drive member (136) which is in engagement with the row of fine teeth (40) is mounted on the fine bearing element (34) in moveable manner, and **in that** the fine bearing element (34) is moveable relative to the base body (36) as a result of a movement of the fine drive member (136) relative to the fine bearing element (34).

7. Surgical retraction device in accordance with claim 6, **characterized in that** the fine drive member (136) is constructed in the form of a fine gear wheel (154).

8. Surgical retraction device in accordance with claim 6 or 7, **characterized in that** the fine drive member (136) is mounted on the fine bearing element (34) such as to be rotatable about a rotational axis of the fine drive member (138) which runs transversely relative to a longitudinal axis (54) of the base body (36)
and/or
**in that** the retraction device comprises a fine actuating element (150) for moving the fine drive member (136) relative to the fine bearing element (34) and/or
**in that** the row of coarse teeth (38) and/or the row of fine teeth (40) respectively extend up to a free end (42, 48) of the base body (36).

9. Surgical retraction device in accordance with any one of the preceding claims, **characterized in that** the at least one fine adjustment mechanism is constructed in the form of a second fine drive mechanism for the purposes of pivoting a support arm relative to the base body.

10. Surgical retraction device in accordance with claim 9, **characterized in that** the second fine drive mechanism (178) is arranged or formed on the coarse bearing element or on the base body.

11. Surgical retraction device in accordance with claim 9 or 10, **characterized in that** the second fine drive mechanism (178) comprises a fine spindle drive (180).

12. Surgical retraction device in accordance with claim 11, **characterized in that** the fine spindle drive (180; 180') comprises a drive spindle (182) which is in contact with a support arm or the coarse bearing element, and **in that** the support arm is pivotal relative to the base body about a pivotal axis as a result of a movement of the drive spindle (182).

13. Surgical retraction device in accordance with any one of the preceding claims, **characterized by** a stop mechanism (192) for limiting a movement of the at least two support arms relative to each other.

14. Surgical retraction device in accordance with any one of the preceding claims, **characterized by** a locking mechanism for locking the at least two support arms (12, 14), which are moveable relative to each other in an adjustment position, in a retention position relative to each other.

15. Surgical retraction device in accordance with any one of the preceding claims, **characterized in that** the support arms (12, 14) are in the form of a straight line or are curved.

## Revendications

1. Dispositif de rétraction chirurgical (10) comprenant au moins deux bras de retenue (12, 14) mobiles l'un par rapport à l'autre et retenus directement ou indirectement l'un sur l'autre, sur lesquels est agencé ou peut être agencé respectivement au moins un élément de retenue (16, 18) chirurgical destiné à retenir des os ou des tissus corporels, et comprenant un système de positionnement de bras de retenue (116) destiné à déplacer et à modifier une position desdits au moins deux bras de retenue (12, 14) l'un par rapport à l'autre, dans lequel le système de positionnement de bras de retenue (116) comporte un système d'ajustement grossier (76) et au moins un système d'ajustement fin (114), au moyen desquels une position desdits au moins deux bras de retenue (12, 14) l'un par rapport à l'autre peut être modifiée indépendamment l'un de l'autre, dans lequel le dispositif de rétractation comporte un corps de base (36) sur lequel au moins l'un desdits au moins deux bras de retenue (12, 14) est retenu mobile, et dans lequel ledit au moins un système d'ajustement fin (114) est réalisé sous la forme d'un premier système d'entraînement fin (118) destiné à déplacer l'un desdits au moins deux bras de retenue (12, 14) et le corps de base (36) l'un par rapport à l'autre,
**caractérisé en ce que** le premier système d'entraînement fin (118) est réalisé sous la forme d'un entraînement fin à crémaillère (120).

2. Dispositif de rétraction chirurgical selon la revendication 1, **caractérisé en ce que** le système d'ajustement grossier (76) et/ou ledit au moins un système d'ajustement fin (114) sont conçus pour rapprocher ou éloigner lesdits au moins deux bras de retenue (12, 14) parallèlement l'un à l'autre.

3. Dispositif de rétraction chirurgical selon la revendication 1 ou 2, **caractérisé en ce que** le système d'ajustement grossier et/ou ledit au moins un système d'ajustement fin sont conçus pour faire pivoter lesdits au moins deux bras de retenue l'un vers l'autre et/ou à distance l'un de l'autre autour d'au moins un axe de pivotement.

4. Dispositif de rétraction chirurgical selon l'une quelconque des revendications précédentes, **caractérisé en ce que** deux bras de retenue ou plus sont retenus mobiles sur le corps de base.

5. Dispositif de rétraction chirurgical selon l'une quelconque des revendications précédentes, **caractérisé en ce que** le système d'ajustement grossier comporte un système d'entraînement grossier (72) destiné à déplacer l'un desdits au moins deux bras de retenue (12, 14) et le corps de base (36) l'un par rapport à l'autre.

6. Dispositif de rétraction chirurgical selon l'une quelconque des revendications précédentes, **caractérisé en ce que** l'entraînement fin à crémaillère (120) comporte une rangée de dents fines (40) sur le corps de base (36) et un élément de support fin (34) monté coulissant sur le corps de base (36), **en ce qu'**un organe d'entraînement fin (136) denté, lequel est engrené avec la rangée de dents fines (40), est monté mobile sur l'élément de support fin (34), et **en ce qu'**à la suite d'un mouvement de l'organe d'entraînement fin (136) par rapport à l'élément de support fin (34), l'élément de support fin (34) est mobile par rapport au corps de base (36).

7. Dispositif de rétraction chirurgical selon la revendication 6, **caractérisé en ce que** l'élément d'entraînement fin (136) est réalisé sous la forme d'une roue dentée fine (154).

8. Dispositif de rétraction chirurgical selon la revendication 6 ou 7, **caractérisé en ce que** l'organe d'entraînement fin (136) est monté sur l'élément de support fin (34) de manière à pouvoir tourner autour d'un axe de rotation d'organe d'entraînement fin (138), lequel s'étend transversalement à un axe longitudinal (54) du corps de base (36)
et/ou
**en ce que** le dispositif de rétraction comporte un élément d'actionnement fin (150) destiné à déplacer l'organe d'entraînement fin (136) par rapport à l'élément de support fin (34)
et/ou
**en ce que** la rangée de dents grossières (38) et/ou la rangée de dents fines (40) s'étendent respectivement jusqu'à une extrémité libre (42, 48) du corps de base (36).

9. Dispositif de rétraction chirurgical selon l'une quelconque des revendications précédentes, **caractérisé en ce que** ledit au moins un système d'ajustement fin est réalisé sous la forme d'un deuxième système d'entraînement fin destiné à faire pivoter un bras de retenue par rapport au corps de base.

10. Dispositif de rétraction chirurgical selon la revendication 9, **caractérisé en ce que** le deuxième système d'entraînement fin (178) est agencé ou formé sur l'élément de support grossier ou sur le corps de base.

11. Dispositif de rétraction chirurgical selon la revendication 9 ou 10, **caractérisé en ce que** le deuxième système d'entraînement fin (178) comporte un mécanisme fin à broche (180).

12. Dispositif de rétraction chirurgical selon la revendication 11, **caractérisé en ce que** le mécanisme fin à broche (180 ; 180') comporte une broche d'entraînement (182), laquelle est en contact avec un bras de retenue ou l'élément de support grossier, et **en ce qu'**à la suite d'un mouvement de la broche d'entraînement (182), le bras de retenue peut pivoter par rapport au corps de base autour d'un axe de pivotement.

13. Dispositif de rétraction chirurgical selon l'une quelconque des revendications précédentes, **caractérisé par** un dispositif de butée (192) destiné à limiter un mouvement desdits au moins deux bras de retenue l'un par rapport à l'autre.

14. Dispositif de rétraction chirurgical selon l'une quelconque des revendications précédentes, **caractérisé par** un dispositif de blocage destiné à bloquer lesdits au moins deux bras de retenue (12, 14), mobiles l'un par rapport à l'autre dans une position d'ajustement, dans une position de retenue.

15. Dispositif de rétraction chirurgical selon l'une quelconque des revendications précédentes, **caractérisé en ce que** les bras de retenue (12, 14) sont rectilignes ou courbés.
